# EUROPÄISCHE PATENTSCHRIFT

(11) **EP 3 742 900 B1**
(45) Veröffentlichungstag und Bekanntmachung des Hinweises auf die Patenterteilung: **23.10.2024**
(21) Anmeldenummer: 19703020.8
(22) Anmeldetag: 23.01.2019
(51) Int. Cl.: G01N 21/15, G01N 33/12, A22C 17/00, A22C 25/00, A22B 5/00, G03B 17/02

(54) **VORRICHTUNG UND ANORDNUNG ZUR OPTISCHEN INSPEKTION VON ARTIKELN DER FISCH- UND FLEISCHVERARBEITENDEN INDUSTRIE**
DEVICE AND ARRANGEMENT FOR OPTICALLY INSPECTING ARTICLES IN THE FISH AND MEAT PROCESSING INDUSTRY
DISPOSITIF ET AGENCEMENT PERMETTANT L'INSPECTION OPTIQUE D'ARTICLES DE L'INDUSTRIE DE TRAITEMENT DU POISSON ET DE LA VIANDE

(30) Priorität: 26.01.2018 DE 102018101835
(43) Veröffentlichungstag der Anmeldung: 02.12.2020
(73) Patentinhaber: NORDISCHER MASCHINENBAU RUD. BAADER GMBH + CO KG, 23560 Lübeck (DE)
(72) Erfinder: PEIN, Roland, 23923 Hamburg (DE)
(74) Vertreter: Stork Bamberger Patentanwälte PartmbB
(86) Internationale Anmeldenummer: PCT/EP2019/051647
(87) Internationale Veröffentlichungsnummer: WO 2019/145367

(56) Entgegenhaltungen:
- WO-A1-2007/090724
- WO-A1-2017/186275
- DE-U1- 202006 019 722
- US-A1- 2013 070 233
- KROMA A/S: "Visiomaster - The good result is now visible", 22 May 2008 (2008-05-22), Internet, XP055334652, Retrieved from the Internet <URL:http://www.odinmakine.com.tr/fileadmin/user_upload/BrosurMakinelerWebSitesi/8-SuurunleriislemeMakineleri/2-KromaAS/Katalog/12-VisionMaster.pdf> [retrieved on 20190113]

## Beschreibung

Die vorliegende Erfindung betrifft eine Vorrichtung, ausgebildet und eingerichtet zur optischen Inspektion von Artikeln der fisch- und fleischverarbeitenden Industrie. Des Weiteren betrifft die Erfindung eine Anordnung zur Bearbeitung und Inspektion der geöffneten Bauchhöhle entweideter Fische, wobei diese eine zum Fördern der Fische in Längsrichtung entlang einer Bearbeitungslinie eingerichtete Fördereinrichtung umfasst.

Derartige Vorrichtungen kommen bei der industriellen Verarbeitung von Fisch- und Fleischwaren zum Einsatz. Die Be- und Verarbeitung derartiger Artikel erfolgt größtenteils mittels automatisch gesteuerter Werkzeuge. Die Verarbeitungsschritte werden zu Kontroll- und Steuerungszwecken optisch überwacht. Hierzu werden die zu bearbeitenden oder bearbeiteten Artikel mittels optischer Bildgeber abgetastet und die so gewonnenen Bilddaten halb- oder vollautomatisch ausgewertet.

Die eingangs genannte Anordnung kommt bei der industriellen Fischverarbeitung zum Einsatz. Hierbei werden die Fische einer automatischen Schlachteinrichtung zugeführt, mittels derer u. a. die Bauchhöhle der Fische geöffnet und die Eingeweide aus dieser durch Eingriff entsprechender Werkzeuge automatisch entfernt werden. Um das Ergebnis dieses Entweidevorgangs zu überprüfen, wird die Bauchhöhle der Fische anschließend optisch abgetastet und auf Basis der erhaltenen Bilddaten mittels geeigneter Bildverarbeitungsalgorithmen entsprechenden Prüfungen durchgeführt.

Bei den bekannten Vorrichtungen und Anordnungen sind die Bildgeber Verunreinigungen ausgesetzt, die während des Verarbeitungsvorgangs entstehenden können. In relativ kurzer Zeit setzen sich beispielsweise Blut- und Gewebsreste, diverse Schmutzpartikel und/oder Flüssigkeitströpfchen an den Objektiven der Bildgeber bzw. in deren Sichtfeld fest. Mit zunehmendem Verunreinigungsgrad wird so nachteiligerweise die freie Sicht des Bildgebers auf die abzutastenden Bereiche der Artikel eingeschränkt, so dass es von Fehlbildauswertungen bis hin zum Totalausfall der optischen Inspektion kommen kann. Der gesamte Produktionsprozess ist in diesem Fall anzuhalten und zunächst eine Reinigung der jeweiligen Komponenten vorzunehmen, so dass es zu unerwünschten Stillstandzeiten und Produktionsunterbrechungen kommt.

Aus der WO 2007/090724 A1 ist eine Vorrichtung zur optischen Inspektion von Artikeln bekannt mit einem lösbar angeordneten Schutzelement.

Es ist daher Aufgabe der vorliegenden Erfindung eine Vorrichtung der eingangs genannten Art vorzuschlagen, die eine möglichst zuverlässige optische Inspektion der genannten Artikel gewährleistet und zugleich möglichst einfach, komfortabel und rasch zu reinigen ist. Des Weiteren besteht die Aufgabe darin, eine Anordnung der eingangsgenannten Art vorzuschlagen, mittels derer eine zuverlässige optische Inspektion der geöffneten Bauchhöhle entweideter Fische ermöglicht wird, die zugleich eine rasche und hygienische Reinigung der für die optische Inspektion relevanten Bauteile erlaubt.

Die Aufgabe wird durch eine Vorrichtung nach Anspruch 1 gelöst. Auf diese Weise ist der Bildgeber einerseits mittels des lichtdurchlässigen Sichtfensters vor unmittelbarer Einwirkung durch Verunreinigung geschützt. Andererseits wird mittels des Schutzelements das Sichtfenster, insbesondere im Erfassungsbereich des Bildgebers, abgeschirmt und der Sicht- bzw. Erfassungsbereich des Bildgebers weitestgehend frei von Verunreinigung gehalten bzw. ein Anhaften von Verunreinigung an dem Sichtfenster stark reduziert.

Ein weiterer Vorteil besteht darin, dass das Schutzelement gehäuseaußenseitig an dem Sichtfenster lösbar angeordnet ist. Zu Reinigungszwecken kann das Schutzelement daher ohne großen Aufwand rasch von dem Sichtfenster gelöst werden und das Sichtfenster und das Schutzelement gereinigt werden. Durch die Abnehmbarkeit des Schutzelements lässt sich dieses insbesondere separat reinigen und - sofern erforderlich - desinfizieren.

Die allseitig gekapselte Anordnung des Bildgebers in dem Gehäuse vereinfacht die Reinigung der erfindungsgemäßen Vorrichtung wesentlich, da der Bildgeber geschützt, insbesondere spritz- und strahlwassergeschützt, angeordnet ist und so auf empfindliche Komponenten des Bildgebers beim Reinigungsvorgang nicht gesondert Rücksicht genommen werden muss.

Unter Hygienegesichtspunkten, an die im Bereich der fisch- und fleischverarbeitenden Industrie regelmäßig besonders hohe Anforderungen gestellt werden, wirkt sich die möglichst einfach gehaltene Struktur der erfindungsgemäßen Vorrichtung besonders positiv aus. Auf diese Weise wird ein andernfalls mögliches Ablagern von Verunreinigungen an unzugänglichen Bereichen weitestgehend ausgeschlossen. Das Gehäuse sowie das Sichtfenster weisen möglichst ebene bzw. plane Oberflächen auf, die besonders leicht und komfortabel zu reinigen sind und im Wesentlichen keine vorsprungs- und/oder hinterschneidungsbehaftete Außengeometrie aufweisen, die ein Anhaften von Verunreinigungen begünstigen und den Reinigungsvorgang insgesamt verkomplizieren würden.

Erfindungsgemäß ist vorgesehen, dass das Schutzelement an dem Sichtfenster angeordnet ist und zumindest einen Erfassungsbereich des Bildgebers unter Beibehaltung freier Sicht auf die Artikel umschließt. Vorteilhafterweise wird so das Sichtfeld des Bildgebers durch das Schutzelement nicht oder nur unwesentlich eingeschränkt. Zugleich ist der Erfassungsbereich des Bildgebers wirksam gegen das Eintreten von beim Verarbeitungsprozess entstehenden Verunreinigungen abgeschirmt. So wird das Auftreffen derartiger Verunreinigungen auf das Sichtfester im relevanten Erfassungsbereich zuverlässig vermieden. Vorzugsweise wird der Erfassungsbereich des Bildgebers von dem Schutzelement allseitig umschlossen. Weiter bevorzugt ist das Schutzelement bezüglich des Bildgebers mittig zentriert angeordnet, so dass die Schirm- und Schutzwirkung des Schutzelements für jeden Teilbereich des Erfassungsbereichs in gleicher Form vorhanden ist. Das Schutzelement ist also vorzugsweise derart angeordnet, dass eine gedachte Mittelachse des Schutzelements mit der Sichtachse des Bildgebers deckungsgleich zusammenfällt.

Erfindungsgemäß weist das Schutzelement eine Schutzelementbasis mit einer zentralen Lichtdurchgangsausnehmung auf und gehäuseseitig sind Haltemittel vorhanden, die jeweils eingerichtet sind, die Schutzelementbasis an dem Sichtfenster lösbar anzuordnen. Die Schutzelementbasis ist als Stütz- und Anlageelement ausgebildet und ist zur Anlage an dem Sichtfenster eingerichtet. Vorzugsweise ist die Schutzelementbasis so ausgebildet, dass diese die Kanalwandung des lichtdurchgängigen Kanals seitlich überragt. Die Schutzelementbasis ist so als Stand- bzw. Anlageflächenerweiterung ausgebildet, mittels derer die Schutzelementbasis an dem Sichtfenster flächig anordenbar eingerichtet ist.

Vorzugsweise ist eine Anlagefläche der Schutzelementbasis plan ausgeführt und liegt im angeordneten Zustand gehäuseaußenseitig an dem Sichtfenster bündig an. Dies bietet den Vorteil, ein unerwünschtes Eindringen von Flüssigkeiten und/oder Verschmutzungen zwischen der Anlagefläche und dem Sichtfenster wirksam zu verhindern.

Erfindungsgemäß sind gehäuseseitig Haltemittel angeordnet, die zur lösbaren Anordnung der Schutzelementbasis an dem Sichtfenster eingerichtet sind. Erfindungsgemäß sind die Haltemittel gehäuseinnenseitig angeordnet. Die Haltemittel sind als Haltemagnete ausgebildet oder umfassen solche. Die Schutzelementbasis weist ebenfalls Haltemagnete auf und/oder ist aus einem magnetischen bzw. magnetisierbaren Werkstoff gefertigt.

Gemäß einer zweckmäßigen Ausgestaltung der Erfindung ist die Schutzelementbasis spaltfrei in die den lichtdurchgängigen Kanal bildende Kanalwandung übergehend eingerichtet. Vorteilhafterweise bilden so die Kanalwandung und die Schutzelementbasis eine durchgängig geschlossene Hüllfläche. Auf diese Weise wird der zu schützende Bereich des Sichtfensters im Erfassungsbereich des Bildsensors optimal vor Verunreinigungen abgeschirmt. Weiter bevorzugt sind die Kanalwandung und die Schutzelementbasis einstückig ausgebildet.

Eine vorteilhafte Weiterbildung der Erfindung sieht vor, dass die Kanalwandung des Schutzelements hohlzylinderförmig mit einer kreisrunden, elliptischen, rechteckigen oder polygonförmigen Querschnittsfläche ausgebildet ist. Durch die Wahl der Querschnittsflächengeometrie kann die Schirmwirkung optimal an den jeweiligen Einsatzzweck angepasst werden. So ist es beispielsweise möglich, bei der Verarbeitung der Artikel der fisch- und fleischverarbeitenden Industrie, bestimmte aus einer Vorzugsrichtung auf die erfindungsgemäße Vorrichtung treffende, Verunreinigungen an den durch die Kanalwandung gebildeten Prallflächen abzuhalten. Je nach Einsatzzweck und der sich daraus ergebenden Hauptrichtung, aus der mit einem Anfall von Verunreinigungen zu rechnen ist, können die von den Kanalwandungen gebildeten Prallflächen entsprechend angepasst werden.

Gemäß einer weiteren vorteilhaften Ausbildung ist die Kanalwandung des Schutzelements als Hohlkegel- oder Hohlpyramidenstumpf ausgebildet. Anders ausgedrückt ist das Schutzelement bzw. die Kanalwandung des Schutzelements trichterartig ausgebildet. Vorzugsweise ist die Seite mit geringerem Querschnitt zu dem Sichtfenster gerichtet, so dass sich der Kanalquerschnitt in Richtung der zu inspizierenden Artikel weitet. Hierdurch wird bei möglichst geringer Baugröße des Schutzelements eine Beeinflussung des optischen Strahlengangs durch das Schutzelement selbst auf ein Minimum reduziert, so dass die Artikel im Erfassungsbereich des Bildgebers ungestört aufgenommen werden können. Die Kanalwandungen sind auf diese Weise optimal an die sich mit zunehmendem Abstand vom Bildgeber gegebene Aufweitung des optischen Strahlengangs angepasst.

Die Aufgabe wird auch durch die eingangs genannte Anordnung gelöst, die sich dadurch auszeichnet, dass diese mindestens eine zur optischen Inspektion der Bauchhöhle eingerichtete Vorrichtung mit zuvor beschriebenen Merkmalen umfasst. Die sich aus der erfindungsgemäßen Vorrichtung ergebenden und zuvor dargelegten Vorteile gelten in gleicher Weise für die erfindungsgemäße Anordnung. Zur Vermeidung von Wiederholung wird daher hierauf verwiesen.

Eine vorteilhafte Weiterbildung zeichnet sich dadurch aus, dass die Fördereinrichtung eingerichtet ist, die Fische mit der geöffneten Bauchhöhle nach oben weisend liegend zu fördern, wobei die mindestens eine Vorrichtung mit dem Sichtfenster zu der Bearbeitungslinie zeigend oberhalb der Bearbeitungslinie angeordnet ist. Dies bietet den Vorteil, dass beim Verarbeitungsprozess entstehende Verunreinigungen der Schwerkraft folgend nach unten abgelenkt werden und bereits hierdurch der Anteil an in den Bereich der erfindungsgemäßen Vorrichtung vordringender Verunreinigung erheblich reduziert ist.

Gemäß einer weiteren Ausbildung ist wenigstens eines der Schutzelemente derart angeordnet, dass der lichtdurchgängige Kanal zumindest im Wesentlichen senkrecht zu der Längsrichtung weisend ausgerichtet ist. Die bei der Verarbeitung der Fische entstehenden festen und flüssigen Bestandteile verteilen sich in alle Raumrichtungen nach dem Zufallsprinzip. Nach oben gerichtete Bewegungskomponenten der Bestandteile werden durch Schwerkrafteinwirkung "gebremst", so dass die Bestandteile sich im Wesentlichen auf mehr oder weniger gekrümmten Bahnen bewegen. Die Wahrscheinlichkeit, dass sich Bestandteile in einer im Wesentlichen senkrecht zum Erdboden gerichteten Bahn entgegen der Schwerkraft bewegen ist folglich deutlich geringer gegenüber anderen Bahnrichtungen. Durch die Ausrichtung des lichtdurchgängigen Kanals senkrecht oder im Wesentlichen senkrecht zu der Längsrichtung ist dieser so ausgerichtet, dass die freie Öffnung des Kanalschenkels in die Richtung zeigt, aus der der geringste Anteil der genannten Bestandteile zu erwarten ist, die zu einer etwaigen Verunreinigung des Sichtfensters führen könnten.

Bevorzugt umfasst die erfindungsgemäße Anordnung mindestens eine zum Beleuchten der Bauchhöhle der Fische eingerichtete Beleuchtungseinrichtung, wobei die Beleuchtungseinrichtung gehäuseinnenseitig an dem Sichtfenster von dem mindestens einen Bildgeber seitlich beabstandet angeordnet ist.

Gemäß einer vorteilhaften Weiterbildung der Erfindung ist vorgesehen, dass die mindestens eine Beleuchtungseinrichtung als Flächenstrahler ausgebildet ist, deren Abstrahlfläche gegenüber dem Sichtfenster geneigt angeordnet ist. Auf diese Weise wird eine möglichst gleichmäßige Ausleuchtung der Bauchhöhle gewährleistet. Zudem wird die Beleuchtungsstärke durch die Überlagerung mehrerer Lichtquellen erhöht.

Eine zweckmäßige Ausbildung zeichnet sich dadurch, dass die Abstrahlfläche so geneigt ist, dass die Hauptlichtabstrahlrichtung jeweils zu einer Sichtlinie zwischen Bildgeber und Bauchhöhle hingeneigt ausgerichtet ist. Durch das Schutzelement entstehende Schattenbildung wird auf diese Weise durch beidseitiges Ausleuchten ausgeschlossen und eine möglichst homogene Ausleuchtung der Bauchhöhle erzielt.

Bevorzugt ist die mindestens eine Beleuchtungseinrichtung unter Bildung eines Luftspaltes an dem Sichtfenster angeordnet. Auf diese Weise ist eine optimale Kühlung der Beleuchtungseinrichtungen und die Einhaltung der maximal zulässigen Betriebstemperatur gewährleistet. Zudem sind die Beleuchtungseinrichtungen auf diese Weise hinterlüftet eingerichtet, was einer andernfalls möglichen Kondensatbildung zwischen den Abstrahlflächen der Beleuchtungseinrichtungen und dem Sichtfenster entgegenwirkt.

Weitere bevorzugte und/oder zweckmäßige Merkmale und Ausgestaltungen der Erfindung ergeben sich aus den Unteransprüchen und der Beschreibung. Besonders bevorzugte Ausführungsformen werden anhand der beigefügten Zeichnung näher erläutert. In der Zeichnung zeigt:
- Fig. 1: eine perspektivische Ansicht der erfindungsgemäßen Vorrichtung,
- Fig. 2: eine Seitenansicht der erfindungsgemäßen Vorrichtung ohne Gehäuse,
- Fig. 3: eine perspektivische Innenansicht der erfindungsgemäßen Vorrichtung mit ausgeblendetem Gehäuse
- Fig. 4: eine schematische Darstellung der erfindungsgemäßen Vorrichtung in Draufsicht mit außenseitigem Blick auf das Sichtfenster und
- Fig. 5: eine perspektivische Ansicht der erfindungsgemäßen Anordnung.

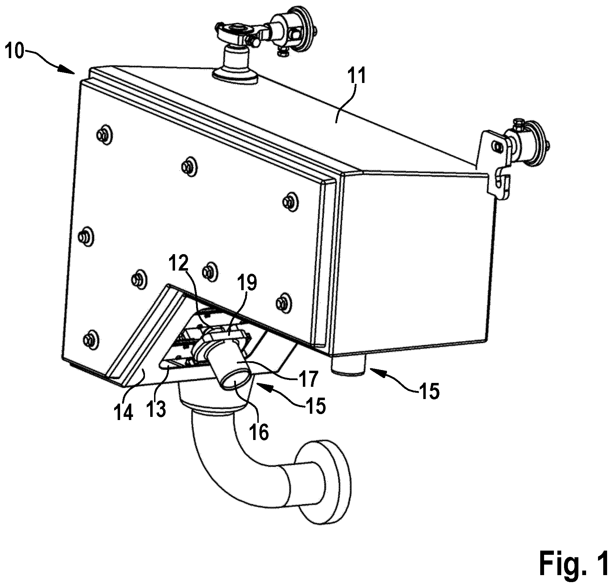

Fig. 1 zeigt eine perspektivische Ansicht der erfindungsgemäßen Vorrichtung 10. Die Vorrichtung 10 ist zur optischen Inspektion von Artikeln der fisch- und fleischverarbeitenden Industrie ausgebildet und eingerichtet. So ist es beispielsweise erforderlich, bei der Verarbeitung von Fischen nach erfolgter Bearbeitung das Bearbeitungsergebnis zu überprüfen, um erforderlichenfalls weitere Nachbearbeitungsschritte einzuleiten. Die gezeigte Vorrichtung 10 umfasst ein Gehäuse 11, in dem ein zum optischen Abtasten der Artikel eingerichteter Bildgeber 12 allseitig gekapselt angeordnet ist. In der Fig. 1 ist nur einer der Bildgeber 12 sichtbar dargestellt, während ein, vorzugsweise in dem Gehäuse 11 angeordneter, zweiter Bildgeber in der Darstellung der Fig. 1 durch das Gehäuse 11 verdeckt wird. Die Zahl der in dem Gehäuse 11 angeordneten Bildgeber 12 ist grundsätzlich beliebig, richtet sich jedoch danach, welche Bereiche der Artikel der fisch- und fleischverarbeitenden Industrie optisch inspiziert werden sollen. Das Gehäuse 11 weist weiter ein lichtdurchlässiges Sichtfenster 13 auf, das vorzugsweise artikelseitig an dem Gehäuse 11 angeordnet ist bzw. an dieser Gehäuseseite 14 eine lichtdurchgängige Ausnehmung bildet.

Gehäuseaußenseitig ist ein Schutzelement 15 an dem Sichtfenster 13 lösbar angeordnet. Das Schutzelement 15 bildet einen lichtdurchgängigen Kanal 16. Ausgehend von dem jeweiligen Bildgeber 12 bildet das Schutzelement 15 mit seinem lichtdurchgängigen Kanal 16 eine freie Sichtachse auf ein Inspektionsbereich, der mittels des jeweiligen Bildgebers zur Inspektion der Artikel optisch abgetastet wird.

Das Schutzelement 15 dient dem Schutz des Sichtfensters 13 vor Verunreinigung, beispielsweise durch Blut- und/oder Gewebsreste sowie vor Flüssigkeitsniederschlag auf dem Sichtfenster 13. Vorzugsweise ist der lichtdurchgängige Kanal 16 des Schutzelements 15 objektivfrei ausgebildet. Mit anderen Worten sind innerhalb des lichtdurchgängigen Kanals 16 insbesondere keine lichtbrechenden optischen Elemente angeordnet, wie beispielsweise Linsen oder optische Filter. Der lichtdurchgängige Kanal 16 wird also vorteilhafterweise ausschließlich durch eine Kanalwandung 17 gebildet, die einerseits als Abschirmelement für das Sichtfenster 13 dienst und dieses gegen die genannten Verunreinigungen entlang der Sichtachse abschirmt und andererseits vorteilhafterweise die Funktion einer Streulichtblende erfüllt. Durch diese wird zusätzlich zu der Schutzfunktion gegen Verunreinigungen des Sichtfensters 13 der Einfall von Licht auf den mindestens einen Bildgeber 12 durch Lichtstrahlen, die nicht im Wesentlichen parallel zu der Sichtachse des Bildgebers 12 verlaufen, wirksam abgeschirmt und gedämpft.

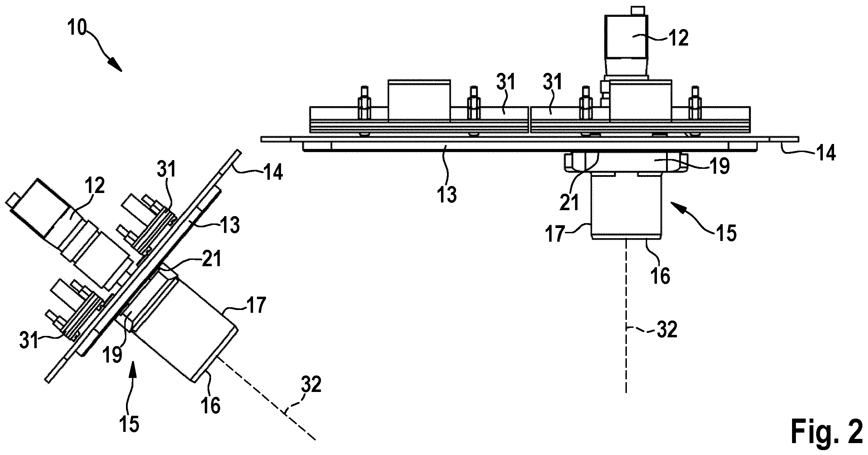

Fig. 2 zeigt eine Seitenansicht der erfindungsgemäßen Vorrichtung, wobei aus Gründen der besseren Übersichtlichkeit nur die Gehäuseseite 14 gezeigt ist. Die beispielhaft in Fig. 2 gezeigte erfindungsgemäße Vorrichtung umfasst zwei der Bildgeber 12, die jeweils geneigt zueinander angeordnet sind, so dass diese die Artikel der fisch- und fleischverarbeitenden Industrie jeweils unter verschiedenen Blickwinkeln inspizieren. Jedem der Bildgeber 12 ist jeweils eines der lichtdurchlässigen Sichtfenster 13 zugeordnet, das wie in der Fig. 2 gezeigt, gemäß einer vorteilhaften Ausbildung der Erfindung zweiteilig ausgebildet ist. Jedem der Bildgeber 12 ist jeweils eines der Schutzelemente 15 zugeordnet, die jeweils gehäuseaußenseitig an dem jeweiligen Sichtfenster 13 lösbar angeordnet sind.

Das Schutzelement 15 ist derart an dem Sichtfenster 13 angeordnet, dass zumindest ein Erfassungsbereich des Bildgebers 12 unter Beibehaltung freier Sicht auf die Artikel umschlossen wird. Der Erfassungsbereich des Bildgebers 12 bezeichnet denjenigen Bereich, innerhalb dessen der Bildgeber 12 in der Lage ist, die Artikel optisch abzutasten. Ausgehend von dem Bildgeber 12 ist so ein Teilbereich des Sichtfensters 13 als zum Erfassungsbereich zugehörigen anzusehen. Das Schutzelement 15 ist nun so um diesen Teilbereich angeordnet, dass dieser von dem Schutzelement 15 einerseits allseitig umschlossen wird, es andererseits jedoch - mit Ausnahme der zuvor beschriebenen Abschirmfunktion gegen Streulicht - zu keiner Einschränkung des bildgeberseitigen Sichtfeldes führt. Vorzugsweise ist das Schutzelement 15 bezüglich des Bildgebers 12 mittig zentriert an dem Sichtfenster 13 angeordnet.

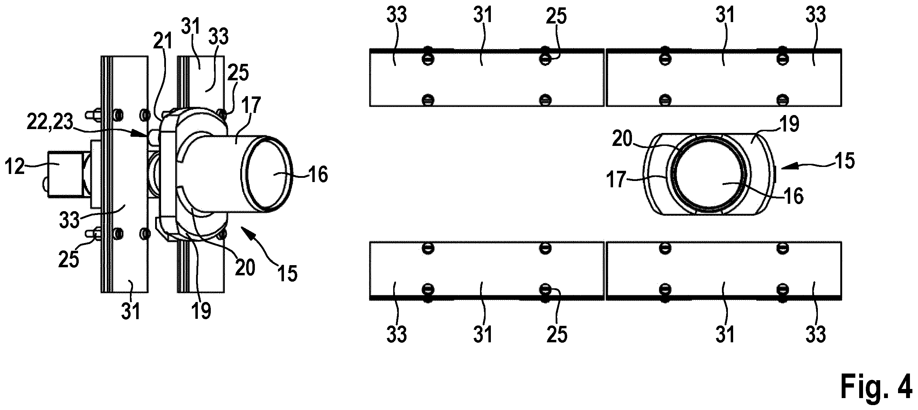

Das Schutzelement 15 weist eine Schutzelementbasis 19 auf. Wie in der Fig. 4 ersichtlich, umfasst die Schutzelementbasis 19 eine zentrale Lichtdurchgangsausnehmung 20. Vorzugsweise ist die Lichtdurchgangsausnehmung 20 fluchtend zu dem lichtdurchgängigen Kanal 16 angeordnet. Die Schutzelementbasis 19 überragt vorzugsweise die Kanalwandung 17 des Schutzelements 15 zumindest seitlich, so dass die Schutzelementbasis 19 eine Anlagefläche 21 bildet, mittels derer die Schutzelementbasis 19 an dem Sichtfenster 13 flächig anordenbar eingerichtet ist.

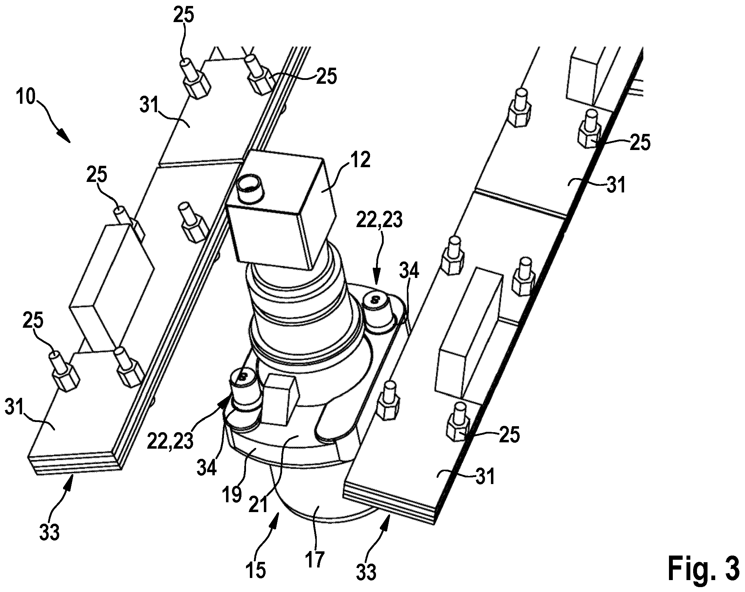

Weiter bevorzugt ist die Anlagefläche 21 plan ausgeführt und liegt im angeordneten Zustand gehäuseaußenseitig an dem Sichtfenster 13 bündig an. Auf diese Weise wird erreicht, dass ein unerwünschtes Eindringen von Flüssigkeiten und/oder Verschmutzungen zwischen der Anlagefläche 21 und dem Sichtfenster 13 ausgeschlossen ist. Gehäuseseitig sind Haltemittel 22 vorhanden, die eingerichtet sind, die Schutzelementbasis 19 an dem Sichtfenster 13 lösbar anzuordnen. Wie in der perspektivischen Innenansicht der erfindungsgemäßen Vorrichtung mit ausgeblendetem Gehäuse in Fig. 3 gezeigt, sind die Haltemittel 22 gehäuseinnenseitig angeordnet. Weiter sind die Haltemittel 22 als Haltemagnete 23 ausgebildet bzw. umfassen jeweils diese Haltemagnete 23.

Die Schutzelementbasis 19 umfasst zumindest teilweise, das heißt zumindest in einigen Teilbereichen, einen magnetischen und/oder magnetisierbaren Werkstoff. Die Schutzelementbasis 19 ist beispielsweise zum Teil oder vollständig aus einem ferromagnetischen Werkstoff hergestellt. Alternativ umfasst die Schutzelementbasis 19 in die Anlagefläche 21 eingelassene Magnete 34 und/oder ferromagnetische Teilflächen. In jedem Fall sind die Polaritäten der Haltemagnete 23 sowie derjenigen der Schutzelementbasis 19 derart ausgerichtet, dass diese sich gegenseitig anziehen und so das gesamte Schutzelement 15 selbstständig an dem Sichtfenster 13 gehalten wird. Ein weiterer Vorteil besteht darin, dass das Schutzelement 15 durch die entgegengesetzten Polaritäten in eine eindeutig zentrierte Anordnung positionierbar ist.

Ein besonderer Vorteil in der zuvor beschriebenen Ausbildung der Haltemittel 22 sowie der Schutzelementbasis 19 besteht in der besonders einfachen Handhabbarkeit der erfindungsgemäßen Vorrichtung. So kann das Schutzelement 15 ohne aufwendige Montagearbeiten, an dem Sichtfenster 13 mit einem einfachen Handgriff angeordnet werden. Zu Reinigungs- und Wartungszwecken lässt sich das erfindungsgemäße Schutzelement 15 in gleicher Weise mit einem einfachen Handgriff von dem Sichtfenster 13 ablösen. Diese wirkt sich insbesondere auf die im Bereich der fisch- und fleischverarbeitenden Industrie regelmäßig vorhandenen hohen Hygieneanforderung aus. Nach Abnahme des Schutzelements 15 von dem Sichtfenster 13 kann das Sichtfenster 13 als planes Bauteil besonders leicht gereinigt werden und es besteht nicht die Gefahr, dass Verunreinigungen aufgrund komplexer Außengeometrien an schlecht zugänglichen Stellen zurückbleiben. Zudem lässt sich das abgenommene Schutzelement 15 separat reinigen und desinfizieren.

Vorteilhafterweise ist die Schutzelementbasis 19 so eingerichtet, dass diese spaltfrei in eine den lichtdurchgängigen Kanal 16 bildende Kanalwandung 17 übergeht. Mit anderen Worten bilden die Kanalwandung 17 und die Schutzelementbasis 19 eine durchgängig geschlossene Hüllfläche, die den lichtdurchgängigen Kanal 16 sowie die Lichtdurchgangsausnehmung 20 abschirmen. Weiter bevorzugt sind die Kanalwandung 17 und die Schutzelementbasis 19 einstückig ausgebildet.

Vorzugsweise ist die Kanalwandung 17 des Schutzelements 15 hohlzylinderförmig ausgebildet. Wie in der Zeichnung gezeigt, weist diese hohlzylinderförmige Ausbildung der Kanalwandung 17 vorzugsweise eine kreisrunde Querschnittsfläche auf. Alternativ ist die Querschnittsfläche beispielsweise elliptisch, rechteckig oder beliebig polygonförmig eingerichtet. Insbesondere bei nicht kreisrunder Querschnittsfläche kann durch Wahl einer entsprechenden Querschnittsflächengeometrie eine verstärkte oder verminderte Schirmwirkung gegen Schmutz und Flüssigkeitseintrag erzielt werden. So ist beispielsweise möglich, bei der Verarbeitung der Artikel der fisch- und fleischverarbeitenden Industrie bestimmte aus einer Vorzugsrichtung auf die erfindungsgemäße Vorrichtung treffende Verunreinigungen an durch die Kanalwandung 17 gebildeten Prallflächen abzuhalten.

Beim Einsatz einer elliptischen Querschnittsfläche lässt sich die Kanalwandung 17 des Schutzelements 15 beispielsweise derart ausrichten, dass die Hauptachse der elliptischen Querschnittsfläche senkrecht oder zumindest im Wesentlichen senkrecht zu der Vorzugsrichtung ausgebildet ist, aus der erwartungsgemäß mit dem größten Anteil an bei der Verarbeitung der Artikel anfallenden Verschmutzung/Verunreinigung zu rechnen ist.

Ergänzend ist die vorliegende Erfindung in Fig. 4 schematisch dargestellt und zeigt die erfindungsgemäße Vorrichtung in Draufsicht mit Blickrichtung auf das Sichtfenster 13. Das Sichtfenster 13 ist in der Fig. 4 ebenso wie das Gehäuse 11 nicht gezeigt.

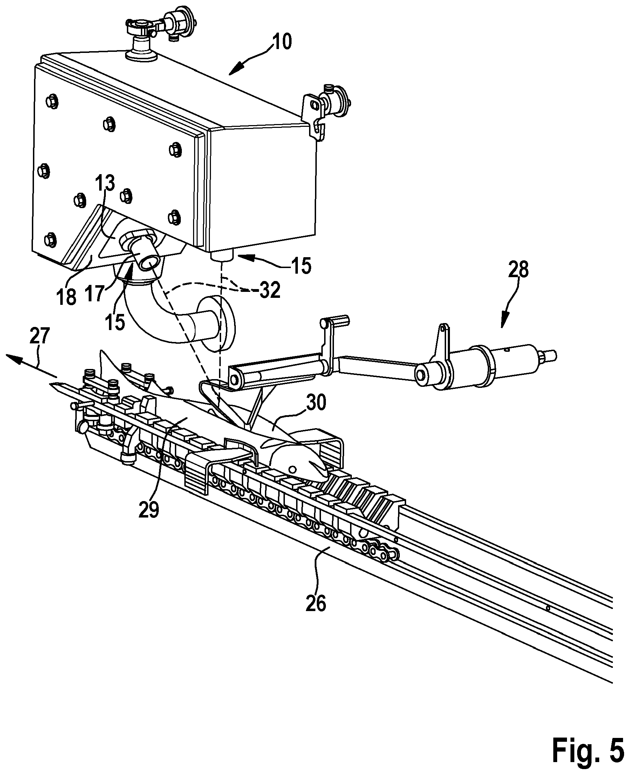

Fig. 5 zeigt eine perspektivische Ansicht der erfindungsgemäßen Anordnung zur Bearbeitung und Inspektion der geöffneten Bauchhöhle 30 entweideter Fische 29. Die erfindungsgemäße Anordnung umfasst eine Fördereinrichtung 26, die zum Fördern der Fische in Längsrichtung 27 entlang einer Bearbeitungslinie 28 eingerichtet ist. Aus Gründen der besseren Übersichtlichkeit ist die Bearbeitungslinie 28 vereinfacht in Form nur eines Werkzeuges zum Offenhalten der Bauchhöhle 30 der Fische dargestellt.

Die Anordnung umfasst weiter mindestens eine zur optischen Inspektion der Bauchhöhle 30 eingerichtete erfindungsgemäße Vorrichtung 10, die zuvor im Detail beschrieben worden ist. Wie der Fig. 5 zu entnehmen ist, ist die erfindungsgemäße Vorrichtung 10 oberhalb der Fördereinrichtung 26 sowie der Bearbeitungslinie 28 angeordnet. Die mittels der Fördereinrichtung 26 transportierten Fische 29 sind rückenseitig auf der Fördereinrichtung 26 gelagert, so dass deren Bauchhöhle 30 nach oben weisend gerichtet ist. Dies bietet den Vorteil, dass bei der Verarbeitung der Fische 29 entstehender Flüssigkeitssprühnebel und/oder Gewebsteile des Fisches 29, die zu einer Verunreinigung des Sichtfensters 13 führen können, schwerkraftbedingt nach unten abgelenkt werden.

Diejenigen Flüssigkeits- oder Gewebsanteile die in die Nähe der erfindungsgemäßen Vorrichtung 10 gelangen, werden wie zuvor beschrieben durch das erfindungsgemäße Schutzelement 15 an einem Auftreffen auf dem Sichtfenster 13 im Bereich des jeweiligen Bildgebers 12 weitestgehend verhindert. Es ist daher besonders vorteilhaft, dass die Fördereinrichtung 26 eingerichtet ist, die Fische 29 mit der geöffneten Bauchhöhle 30 nach oben weisend liegend zu fördern und die mindestens eine Vorrichtung 10 mit dem Sichtfenster 13 zur Bearbeitungslinie 28 zeigend oberhalb der Bearbeitungslinie 28 anzuordnen.

Vorzugsweise ist zumindest eines der Schutzelemente 15 derart angeordnet, dass der lichtdurchgängige Kanal 16 zumindest im Wesentlichen senkrecht zu der Längsrichtung 27 ausgerichtet ist. Wie in Fig. 5 gezeigt, ist eines der Schutzelemente 15, das bezüglich der Längsrichtung 27 vorgeordnet ist, exakt senkrecht gegenüber der Längsrichtung 27 angeordnet. Das weitere nachgeordnete der Schutzelemente 15 ist gegenüber der Längsrichtung 27 geneigt angeordnet, weicht also etwas von der senkrechten Ausrichtung ab.

Wie in Fig. 2, 3 und 4 gezeigt, umfasst die erfindungsgemäße Anordnung mindestens eine zum Beleuchten der Bauchhöhle 30 der Fische 29 eingerichtete Beleuchtungseinrichtung 31. Jede der Beleuchtungseinrichtungen 31 ist vorzugsweise gehäuseinnenseitig an dem Sichtfenster 13 angeordnet und jeweils von dem mindestens einen Bildgeber 12 seitlich beabstandet. Wie in Fig. 4 gezeigt, sind beispielsweise um das Schutzelement 15 vier der Beleuchtungseinrichtungen 31 seitlich beabstandet angeordnet. Seitlich neben den links in Fig. 4 gezeigten Bildgeber 12 sind jeweils zwei der Beleuchtungseinrichtungen 31 angeordnet.

Vorteilhafterweise ist die mindestens eine Beleuchtungseinrichtung 31 als Flächenstrahler ausgebildet. Die Beleuchtungseinrichtung 31 umfasst hierzu beispielsweise LED-Arrays. Weiter bevorzugt ist deren Abstrahlfläche 33 gegenüber dem Sichtfenster 15 geneigt angeordnet, insbesondere ist die Abstrahlfläche 33 so geneigt, dass die Hauptlichtabstrahlrichtung jeweils zu einer der Sichtlinien 32 zwischen dem jeweiligen der Bildgeber 12 und der Bauchhöhle 30 hingeneigt ausgerichtet ist. Hierdurch wird einerseits die Beleuchtungsstärke durch Überlagerung der Lichtanteile der Beleuchtungseinrichtungen 31 vergrößert. Anderseits wird durch die beidseits des Schutzelements 15 angeordneten Beleuchtungseinrichtungen 31 eine gleichmäßige Ausleuchtung der Bauchhöhle 30 gewährleistet und etwaige Verschattungen durch das Schutzelement 15 selbst vermieden.

Die gezeigten Beleuchtungseinrichtungen 31 sind jeweils unter Bildung eines Luftspaltes an dem Sichtfenster 15 angeordnet ist. Diese Hinterlüftung der Beleuchtungseinrichtungen 31 wirkt sich positiv auf Abfuhr entstehender Abwärme aus und bietet zugleich den Vorteil, dass mögliche Kondensatbildung zwischen dem Sichtfenster 13 und der Abstrahlfläche 33 vermieden wird.

Die Beleuchtungseinrichtungen 31 sind vorzugsweise mittels Schraubverbindungen 25 an dem Sichtfenster 13 bzw. an der Gehäuseseite 14 lösbar angeordnet.

## Patentansprüche

1. Vorrichtung (10), ausgebildet und eingerichtet zur optischen Inspektion von Artikeln der fisch- und fleischverarbeitenden Industrie, umfassend
mindestens einen in einem Gehäuse (11) allseitig gekapselt angeordneten und zum optischen Abtasten der Artikel eingerichteten Bildgeber (12), wobei
das Gehäuse (11) ein lichtdurchlässiges Sichtfenster (13) aufweist und
gehäuseaußenseitig ein einen lichtdurchgängigen Kanal (16) bildendes Schutzelement (15) zum Schutz des Sichtfensters (13) vor Verunreinigen lösbar angeordnet ist und, dass das Schutzelement (15) an dem Sichtfenster (13) angeordnet ist und zumindest einen Erfassungsbereich des Bildgebers (12) unter Beibehaltung freier Sicht auf die Artikel umschließt, wobei
das Schutzelement (15) eine Schutzelementbasis (19) mit einer zentralen Lichtdurchgangsausnehmung (20) aufweist und gehäuseseitig Haltemittel (22) vorhanden sind, die jeweils eingerichtet sind, die Schutzelementbasis (19) an dem Sichtfenster (13) lösbar anzuordnen, und wobei
die Haltemittel (22) gehäuseinnenseitig angeordnet und als Haltemagneten (23) ausgebildet sind und die Schutzelementbasis (19) zumindest teileweise einen magnetischen und/oder magnetisierbaren Werkstoff umfasst.

2. Vorrichtung nach Anspruch 1, **dadurch gekennzeichnet, dass** die Schutzelementbasis (19) spaltfrei in eine den lichtdurchgängigen Kanal (16) bildende Kanalwandung (17) übergehend eingerichtet ist.

3. Vorrichtung nach Anspruch 2, **dadurch gekennzeichnet, dass** die Kanalwandung (17) und die Schutzelementbasis (19) einstückig ausgebildet ist.

4. Vorrichtung nach einem der Ansprüche 2 oder 3, **dadurch gekennzeichnet, dass** die Kanalwandung (17) des Schutzelements (15) hohlzylinderförmig mit einer kreisrunden, elliptischen, rechteckigen oder polygonförmigen Querschnittsfläche ausgebildet ist.

5. Vorrichtung nach einem der Ansprüche 2 oder 3, **dadurch gekennzeichnet, dass** die Kanalwandung (17) des Schutzelements (15) als Hohlkegel- oder Hohlpyramidenstumpf ausgebildet ist.

6. Anordnung zur Bearbeitung und Inspektion der geöffneten Bauchhöhle (30) entweideter Fische (29), umfassend
eine zum Fördern der Fische (29) in Längsrichtung (27) entlang einer Bearbeitungslinie (28) eingerichtete Fördereinrichtung (26),
**dadurch gekennzeichnet,**
**dass** die Anordnung mindestens eine zur optischen Inspektion der Bauchhöhle (30) eingerichtete Vorrichtung (10) nach einem der Ansprüche 1 bis 5 umfasst.

7. Anordnung nach Anspruch 6, **dadurch gekennzeichnet, dass** die Fördereinrichtung (26) eingerichtet ist, die Fische (29) mit der geöffneten Bauchhöhle (30) nach oben weisend liegend zu fördern, wobei die mindestens eine Vorrichtung (10) mit dem Sichtfenster (13) zu der Bearbeitungslinie (28) zeigend oberhalb der Bearbeitungslinie (28) angeordnet ist.

8. Anordnung nach Anspruch 7, **dadurch gekennzeichnet, dass** wenigstens eines der Schutzelemente (15) derart angeordnet ist, dass der lichtdurchgängige Kanal (16) zumindest im Wesentlichen senkrecht zu der Längsrichtung (27) weisend ausgerichtet ist.

9. Anordnung nach einem der Ansprüche 6 bis 8weiter umfassend mindestens eine zum Beleuchten der Bauchhöhle (30) der Fische (29) eingerichtete Beleuchtungseinrichtung (31), wobei die Beleuchtungseinrichtung (31) gehäuseinnenseitig an dem Sichtfenster (13) von dem mindestens einen Bildgeber (12) seitlich beabstandet angeordnet ist.

10. Anordnung nach einem der Ansprüche 6 bis 9, **dadurch gekennzeichnet, dass** die mindestens eine Beleuchtungseinrichtung (31) als Flächenstrahler ausgebildet ist, deren Abstrahlfläche (33) gegenüber dem Sichtfenster (13) geneigt angeordnet ist.

11. Anordnung nach Anspruch 10, **dadurch gekennzeichnet, dass** die Abstrahlfläche (33) so geneigt ist, dass die Hauptlichtabstrahlrichtung jeweils zu einer Sichtlinie (32) zwischen Bildgeber (12) und Bauchhöhle (30) hingeneigt ausgerichtet ist.

12. Anordnung nach einem der Ansprüche 9 bis 11, **dadurch gekennzeichnet, dass** die mindestens eine Beleuchtungseinrichtung (31) unter Bildung eines Luftspaltes an dem Sichtfenster (13) angeordnet ist.

## Claims

1. Apparatus (10) designed and configured for optically inspecting articles in the fish and meat processing industry, comprising
at least one imaging system (12) which is arranged so as to be encapsulated on all sides in a housing (11) and is configured for optically scanning the articles, wherein
the housing (11) has a transparent viewing window (13) and
a protective element (15), forming a light-transmissive channel (16), is releasably arranged on the outside of the housing to protect the viewing window (13) from contamination, and
that the protective element (15) is arranged on the viewing window (13) and encloses at least one detection zone of the imaging system (12) while maintaining an unobstructed view of the articles,
the protective element (15) comprises a protective element base (19) with a central light transmittance recess (20) and holding means (22), which are configured in each case to arrange the protective element base (19) releasably on the viewing window (13), are present on the housing side, and wherein
the holding means (22) are arranged on the inside of the housing and are designed as holding magnets (23) and the protective element base (19) comprises at least in part a magnetic and/or magnetisable material.

2. Apparatus according to claim 1, **characterised in that** the protective element base (19) is configured to merge gap-free into a channel wall (17) forming the light-transmissive channel (16).

3. Apparatus according to claim 2, **characterised in that** the channel wall (17) and the protective element base (19) are formed integrally.

4. Apparatus according to one of claims 2 or 3, **characterised in that** the channel wall (17) of the protective element (15) is shaped as a hollow cylinder with a circular, elliptical, rectangular or polygonal cross-sectional area.

5. Apparatus according to one of claims 2 or 3, **characterised in that** the channel wall (17) of the protective element (15) is designed as a hollow truncated cone or hollow truncated pyramid.

6. Arrangement for processing and inspecting the opened abdominal cavity (30) of gutted fish (29), comprising
a conveying device (26) configured for conveying the fish (29) along a processing line (28) in the longitudinal direction (27),
**characterised in that**,
the arrangement comprises at least one apparatus (10) configured for optically inspecting the abdominal cavity (30) according to any one of claims 1 to 5.

7. Arrangement according to claim 6, **characterised in that** the conveying device (26) is configured to convey the fish (29) lying with the opened abdominal cavity (30) pointing upwards, wherein the at least one apparatus (10) is arranged above the processing line (28) with the viewing window (13) pointing towards said processing line (28).

8. Arrangement according to claim 7, **characterised in that** at least one of the protective elements (15) is arranged in such a manner that the light-transmissive channel (16) is oriented at least substantially perpendicular to the longitudinal direction (27).

9. Arrangement according to any one of claims 6 to 8, further comprising at least one illumination device (31) configured for illuminating the abdominal cavity (30) of the fish (29), wherein the illumination device (31) is arranged on the viewing window (13) on the inside of the housing spaced apart laterally from the at least one imaging system (12).

10. Arrangement according to any one of claims 6 to 9, **characterised in that** the at least one illumination device (31) is designed as surface-emitting diode, the radiating surface (33) of which is arranged sloping with respect to the viewing window (13).

11. Arrangement according to claim 10, **characterised in that** the radiating surface (33) is inclined such that in each case the main light emission direction is oriented sloping towards a viewing line (32) between imaging system (12) and abdominal cavity (30).

12. Arrangement according to any one of claims 9 to 11, **characterised in that** the at least one illumination device (31) is arranged on the viewing window (13), forming an air gap.

## Revendications

1. Dispositif (10), configuré et adapté pour l'inspection optique d'articles de l'industrie de transformation du poisson et de la viande, comprenant
au moins un imageur (12) agencé dans un boîtier (11) encapsulé de tous côtés et adapté pour le balayage optique des articles,
le boîtier (11) présentant une fenêtre d'observation (13) translucide et
un élément de protection (15) formant un canal (16) laissant passer la lumière étant agencé de manière amovible sur le côté extérieur du boîtier pour protéger la fenêtre d'observation (13) contre les saletés, et l'élément de protection (15) étant agencé sur la fenêtre d'observation (13) et entourant au moins une zone de détection de l'imageur (12) en conservant une vue libre sur les articles,
l'élément de protection (15) présentant une base d'élément de protection (19) avec un évidement de passage de lumière central (20) et des moyens de maintien (22) étant présents côté boîtier, lesquels sont respectivement adaptés pour agencer de manière amovible la base d'élément de protection (19) sur la fenêtre d'observation (13), et
les moyens de maintien (22) étant agencés sur le coté intérieur du boîtier et étant configurés sous forme d'aimants de maintien (23) et la base d'élément de protection (19) comprenant au moins en partie un matériau magnétique et/ou magnétisable.

2. Dispositif selon la revendication 1, **caractérisé en ce que** la base d'élément de protection (19) est adaptée de manière à se fondre sans fente dans une paroi de canal (17) formant le canal (16) laissant passer la lumière.

3. Dispositif selon la revendication 2, **caractérisé en ce que** la paroi de canal (17) et la base d'élément de protection (19) sont configurées d'une seule pièce.

4. Dispositif selon l'une quelconque des revendications 2 ou 3, **caractérisé en ce que** la paroi de canal (17) de l'élément de protection (15) est configurée sous forme cylindrique creuse avec une surface de section transversale circulaire, elliptique, rectangulaire ou polygonale.

5. Dispositif selon l'une quelconque des revendications 2 ou 3, **caractérisé en ce que** la paroi de canal (17) de l'élément de protection (15) est configurée sous forme de cône creux ou de pyramide creuse tronquée.

6. Agencement de traitement et d'inspection de la cavité abdominale ouverte (30) de poissons éviscérés (29), comprenant
un appareil de transport (26) adapté pour transporter les poissons (29) dans la direction longitudinale (27) le long d'une ligne de traitement (28),
**caractérisé en ce que**
l'agencement comprend au moins un dispositif (10) adapté pour l'inspection optique de la cavité abdominale (30) selon l'une quelconque des revendications 1 à 5.

7. Agencement selon la revendication 6, **caractérisé en ce que** l'appareil de transport (26) est adapté pour transporter les poissons (29) couchés avec la cavité abdominale ouverte (30) orientée vers le haut, l'au moins un dispositif (10) étant agencé au-dessus de la ligne de traitement (28) avec la fenêtre d'observation (13) orientée vers la ligne de traitement (28).

8. Agencement selon la revendication 7, **caractérisé en ce qu'**au moins l'un des éléments de protection (15) est agencé de telle sorte que le canal (16) laissant passer la lumière est orienté au moins essentiellement perpendiculairement à la direction longitudinale (27).

9. Agencement selon l'une quelconque des revendications 6 à 8, comprenant en outre au moins un appareil d'éclairage (31) adapté pour éclairer la cavité abdominale (30) des poissons (29), l'appareil d'éclairage (31) étant agencé sur le côté intérieur du boîtier sur la fenêtre d'observation (13), à distance latérale de l'au moins un imageur (12).

10. Agencement selon l'une quelconque des revendications 6 à 9, **caractérisé en ce que** l'au moins un appareil d'éclairage (31) est configuré sous forme de projecteur de surface dont la surface de rayonnement (33) est agencée inclinée par rapport à la fenêtre d'observation (13).

11. Agencement selon la revendication 10, **caractérisé en ce que** la surface de rayonnement (33) est inclinée de telle sorte que la direction principale de rayonnement lumineux est orientée de manière inclinée par rapport à une ligne de visée (32) entre l'imageur (12) et la cavité abdominale (30).

12. Agencement selon l'une quelconque des revendications 9 à 11, **caractérisé en ce que** l'au moins un appareil d'éclairage (31) est agencé sur la fenêtre d'observation (13) en formant un entrefer.
